# EUROPÄISCHE PATENTSCHRIFT

(11) **EP 1 828 665 B1**
(45) Veröffentlichungstag und Bekanntmachung des Hinweises auf die Patenterteilung: **17.04.2013**
(21) Anmeldenummer: 05821588.0
(22) Anmeldetag: 23.12.2005
(51) Int. Cl.: F16L 59/02, F16L 59/08

(54) **DÄMMELEMENT**
INSULATING ELEMENT
ELEMENT ISOLANT

(30) Priorität: 23.12.2004 DE 202004020172 U; 14.05.2005 DE 202005007653 U
(43) Veröffentlichungstag der Anmeldung: 05.09.2007
(73) Patentinhaber: Thermamax Hochtemperaturdämmungen GmbH, 68169 Mannheim (DE)
(72) Erfinder: BECKER, Michael, 67336 Weingarten (DE)
(74) Vertreter: Schmitt, Meinrad
(86) Internationale Anmeldenummer: PCT/EP2005/013997
(87) Internationale Veröffentlichungsnummer: WO 2006/069756

(56) Entgegenhaltungen:
- BE-A3- 1 014 626
- DE-U1-202005 015 340
- FR-A- 2 847 650
- US-B1- 6 276 044

## Beschreibung

Die Erfindung bezieht sich auf ein Dämmelement gemäß den im Oberbegriff des Patentanspruchs angegebenen Merkmalen.

Aus der BE 1 014 626 A3 ist ein derartiges Dämmelement bekannt, welches als eine Struktur ausgebildet ist und mehrere Lagen von Edelstahlfolien enthält. Zwischen den - wie das gesamte Dämmelement - strukturierten Edeistahifolien können weitere Lagen aus Fasermaterial sowie Klebemittel vorgesehen sein. Zur Ausbildung der Edelstahlfolien mit zueinander beabstandet angeordneten Mikrostrukturen und zum Einsatz eines Hochtemperaturklebers zur Verbindung des Fasermaterials mit der Edelstahlfolie sind der BE 1 014 626 A3 keine Hinweise zu entnehmen.

Des Weiteren sind aus der US 6 276 044 B1 Dämmelemente bekannt, deren Edelstahlfolien mit Strukturen versehen sind. Es handelt sich hierbei um aus mehreren Lagen von Edelstahlfolien aufgebaute Dämmelemente, wobei die Strukturen der jeweiligen Edelstahlfolie vergleichsweise große Abmessungen aufweisen. Mittels der genannten Strukturen werden zwischen den einzelnen Lagen bzw. Edelstahlfolien recht große Abstände vorgegeben, um ein entsprechend großes Luftvolumen zwecks Wärmedämmung vorzugeben.

Ferner ist aus der DE 198 25 813 C2 ein Dämmelement bekannt, welches als eine flexible, mehrlagige Metallfolienstruktur mit mindestens zwei Lagen aus Metallfolien mit einer Dicke von jeweils 0,15 mm oder weniger ausgebildet sind. Die beiden Metallfolieneinlagen sind gewellt und stapelförmig ineinander greifend angeordnet, wobei ein Abschnitt der Wellen des Stapels zusammengedrückt ist, um sich gegenseitig verblockende Falten der Lagen zu bilden. Zwischen den Metall-Lagen können andere Materiallagen angeordnet sein, wie beispielsweise Kunststoff-Filme oder Fasermaterialien, um die schalldämpfenden Eigenschaften zu verbessen. Des Weiteren können die Metall-Lagen geprägt sein oder mit Buckeln, Vertiefungen, Falten oder Kräuselungen versehen sein, um Zwischenräume und Abstände zwischen den genannten Metall-Lagen zu erhalten oder die wärmedämmenden oder schallisolierenden Eigenschaften des Dämmelements zu verbessern.

Weiterhin ist aus der DE 35 21 467 A1 eine wärmedämmende Ummantelung eines Rohres zur Leitung von Heißgasen, insbesondere zur Abgasrückführung in Diesel-Personenkraftwagen bekannt, welche aus einem den Außenumfang des Rohres umgebenden Keramikfasermaterial und einer darauf angeordneten, das Keramikfasermaterial zusammen haltendenden Metallfolie besteht. Die Metallfolie kann als eine Edelstahlfolie mit einer Dicke von ca. 0,1 mm ausgebildet sein. Das Keramikfasermaterial wird bei der Montage bevorzugt als Formteilhälften auf der Außenseite des zu isolierenden Rohres angeordnet und nachfolgend wird die Metallfolie außen um das Keramikfasermaterial herum gelegt, wobei die Längskanten der Metallfolie in geeigneter Weise, wie beispielsweise durch Schweißen, miteinander verbunden werden. Der diesbezügliche Fertigungs- und Montageaufwand ist nicht unerheblich.

Des Weiteren sind Dämmelemente in Form von Fasermatten aus Glasfasern oder Mineralfasern bekannt, welche vor allem für Isolieranwendungen, wie beispielsweise zur Motorraumdämmung in Nutzfahrzeugen, Innenverkleidung von Turboladerisolationen, Innenverkleidung von Abgasverkleidungen usw., zum Einsatz gelangen. Derartige Dämmelemente sind mit einer Aluminiumbeschichtung versehen bzw. mit einer Aluminiumfolie kaschiert, wobei zur Verbindung mit der Fasermatte ein Klebemittel vorgesehen ist. Diese Dämmelemente eignen sich zwar für vergleichsweise niedrige Temperaturbereiche von einigen 100°C, wobei vor allem die Temperaturbelastbarkeit des Klebemittels im Bereich von 150 bis 200°C Grenzen hinsichtlich höherer Temperaturbereiche setzt. Des Weiteren sind die konträren Anforderungen hinsichtlich Verformbarkeit einerseits und Formstabilität andererseits ebenso zu beachten wie die Temperaturbeständigkeit des Dämmelements bei höheren Temperaturen.

Hiervon ausgehend liegt der Erfindung die Aufgabe zugrunde, das Dämmelement der eingangs genannten Art dahingehend weiterzubilden, dass mit einem geringen Fertigungs- und Materialaufwand den Anforderungen hinsichtlich erhöhter Temperaturbelastungen entsprochen werden kann. Das Dämmelement soll mit herkömmlichen Maschinen problemlos gefertigt werden können und insbesondere für Hochtemperaturanwendungen eine lange Lebensdauer und/oder große Formbeständigkeit aufweisen. Ferner soll das Dämmelement ohne Schwierigkeiten den jeweiligen Temperaturdämmanforderungen entsprechend optimiert werden können.

Die Lösung dieser Aufgabe erfolgt gemäß den im Patentanspruch 1 angegebenen Merkmalen.

Das erfindungsgemäße Dämmelement zeichnet sich vor allem durch eine hohe Formstabilität aus, wobei gleichwohl eine einfache Handhabung bei der Verarbeitung und/oder eine gute Verformbarkeit zwecks Anpassung an die jeweiligen Einsatzbedingungen gegeben ist. Das Dämmelement enthält eine dünne Edelstahlfolie mit einer vorgegebenen Mikrostrukturierung, wodurch optimierte Eigenschaften in Bezug auf thermische Belastungen, insbesondere im Bereich von 1.200°C, ebenso wie dynamischen Belastungen erreicht werden. Durch die Mikrostrukturen ist die Flexibilität der Edelstahlfolie, im Vergleich mit einer Edelstahlfolie ohne Mikrostrukturen, erheblich und/oder um einen vorgegebenen Faktor vergrößert, wodurch die Anpaßbarkeit des Dämmelements an die Geometrie des zu dämmenden Bauteils, wie Abgasrohre, Motorteile oder sonstige Maschinenelemente, bei der Montage nicht unerheblich verbessert wird.

Die Mikrostrukturen werden insbesondere durch Prägen der zunächst ebenen Edelstahlfolie in diese eingebracht, und zwar vor dem Verbinden mit der Fasermatte. Das erfindungsgemäße Dämmelement enthält nur eine einzige mit den genannten Mikrostrukturen versehene Edelstahlfolie, welche nur auf die eine Oberfläche der flexiblen Fasermatte aufkaschiert ist, wobei zur Verbindung ein Klebemittel vorgesehen ist. Bei Blickrichtung auf das mit der mikrostrukturierten Edelstahlfolie versehene Dämmelement sind die Mikrostrukturen als zur Fasermatte gerichtete Täler oder als von der Fasermatte nach außen gerichtete Erhebungen ausgebildet. Die Edelstahlfolie weist auf ihrer der flexible Fasermatte zugewandten Innenseite somit Innenbereiche auf, welche zwischen den Tälern oder in den Erhebungen sich befinden. Die Innenbereiche sind zumindest teilweise mit dem Klebemittel ausgefüllt, welches weiterhin teilweise in eine äußere Grenzschicht der Fasermatte eingedrungen ist. Außer der Adhäsionsbindung des Klebemittels mit der Edelstahlfolie ist somit eine zahnartige und formschlüssige ineinander greifende Verbindung des Klebemittels und der das Klebemittel enthaltenden Grenzschicht der Fasermatte mit der Edelstahlfolie vorhanden, wodurch eine erheblich verbesserte formschlüssige Verbindung der Edelstahlfolie mit der Fasermatte aufgrund des in die genannten Innenbereiche eingreifender Klebemittels erreicht ist.

Die Mikrostrukturierung der Edelstahlfolie ist derart vorgegeben, dass die quer zur Ebene der Edelstahlfolie gemessenen Abstände zwischen den außen liegenden Spitzenbereichen und den innen liegenden Talbereichen der Mikrostrukturen um einen vorgegebenen Faktor erheblich größer sind als die Materialdicke der Folie, wobei die Materialdicke im Bereich zwischen 0,06 bis 0,15 mm, bevorzugt im Bereich zwischen 0,07 bis 0,13 mm und insbesondere im Bereich zwischen 0,09 bis 0,12 mm vorgegeben ist. Aufgrund der erfindungsgemäßen Ausbildung der Materialdicke ist die Edelstahlfolie problemlos mit herkömmlichen Maschinen, insbesondere einem CNC Cutter, verarbeitbar. Die Mikrostrukturen sind in vorgegebenen kleinen Abständen zueinander und/oder dicht nebeneinander angeordnet. Die Abstände zwischen den Mikrostrukturen, welche bevorzugt als Prägungen in die Edelstahlfolie eingebracht sind, betragen überwiegend maximal 3 mm, zweckmäßig 2 mm und insbesondere im wesentlichen 1 mm. Aufgrund der erfindungsgemäß dicht nebeneinander angeordneten Mikrostrukturen besitzt, im Vergleich mit einer weitgehend planen Edelstahlfolie, die erfindungsgemäße Edelstahlfolie eine erheblich verbesserte Stabilität, wobei aufgrund der vorgegebenen geringen Materialdicke gleichwohl eine einfache Bearbeitung mit herkömmlichen Maschinen, insbesondere das Zuschneiden auf die der jeweiligen Verwendung entsprechenden Abmessungen, des gesamten Dämmelements einschließlich der Fasermatte nach deren Verbindung mit der Edelstahlfolie problemlos durchführbar ist.

Die Edelstahlfolie besteht vorteilhaft aus einer Eisen-Chrom-Nickel-Legierung und enthält bedarfsweise geringe Gewichtsprozente von Silizium und/oder Mangan und/oder Tungsten und/oder Aluminium und/oder Columbium und/oder Titan und/oder Kupfer. Bevorzugt liegt der Eisenanteil in Gew.% bei 50 bis 80%, der Chromanteil zwischen 10 bis 28% und der Nickelanteil zwischen 5 bis 25%, gegebenenfalls in Kombination mit wenigstens einem der vorstehend genannten Zuschlagsstoffe. Das Dämmelement zeichnet sich durch eine optimierte Wärmedämmung vor allem im Hochtemperaturbereich ebenso aus wie durch eine verbesserte Schalldämmung.

Zur Verbindung der mikrostrukturierten Edelstahlfolie mit der flexiblen Fasermatte ist als Klebemittel ein Hochtemperaturkleber vorgesehen, welcher sich durch eine Temperaturbeständigkeit bis zu 500°C auszeichnet. Es gelangt ein nicht brennbarer und wasserfester Dispersionskleber zum Einsatz, welcher zur Kaschierung von nicht bzw. schwer entflammbaren Materialien geeignet ist, wie die Fasermatte aus Mineralwolle und/oder Mineralfasern sowie aus Glasfasern undloder Glasvlies, um somit die Klebeverbindung mit der Edelstahlfolie dauerhaft sicherzustellen. Das erfindungsgemäß zum Einsatz gelangende Klebemittel, und zwar der Dispersionkleber, besteht im wesentlichen aus einer wässrigen Kunststoffdispersion mit Zusätzen von Alkalisilikaten, Wasser abspaltenden Flammschutzmitteln und Additiven zur Eigenschaftsverbesserung. Das Klebemittel ist in vorteilhafter Weise frei von organischen Lösungsmitteln, wobei ein vorteilhaft Wasser abspaltendes Flammschutzmittel zweckmäßig frei von Halogenen und Antimontrioxid ausgebildet ist. Bei der Untersuchung des Brandverhaltens nach DIN 4102 T1 wurden je nach eingesetztem Basissubstrat bzw. Fasermatte und Einsatz des genannten Dispersionsklebers zur Kaschierung die Baustoffklasse A1 erreicht, wobei als Auftragsmenge 100 g Dispersionskleber (naß) /m² vorgesehen waren. Da der Dispersionskleber frei von organischen Lösungsmitteln ist, wird zwar eine längere Trocknungszeit benötigt, doch werden andererseits bei der Herstellung ein aufwendiges Absaugen sowie Brand- und Explosionsvorkehrungen in vorteilhafter Weise vermieden. Darüber hinaus sei festgehalten, dass die Trocknungszeit in bevorzugter Weise durch weitere Maßnahmen, wie insbesondere Aufsprühen des Klebemittels undloder Umluft oder Infrarottrocknung erheblich reduziert wird. Schließlich enthält in einer vorteilhaften Weiterbildung das Klebemittel toxisch unbedenkliche Flammschutzmittel, wobei insbesondere auf die Freiheit von Chlor- und Bromverbindungen sowie Antimontrioxid hingewiesen sei.

Die Fasermatte ist in vorteilhafter Weise als Fasernadelmatte ausgebildet und enthält zweckmäßig zum einen Glasfasern und zum anderen Mineralfasern. Es hat sich als zweckmäßig erwiesen, Silikatfasern mit einem Gewichtsanteil im Bereich zwischen 20 bis 40% und Basaltfasern im Bereich zwischen 80 bis 60% vor zu sehen, wobei ein Gewichtsanteil der Silikatfasern von zumindest näherungsweise 30% und ein Gewichtsanteil der Basaltfasern von zumindest näherungsweise 70% sich als besonders vorteilhaft erwiesen hat. Die Fasern, insbesondere die Silikatfasern, besitzen eine Faserstärke im Mikrometerbereich, vorteilhaft im Bereich zwischen 10 bis 17 µm. Bei einer Nenndicke der Fasermatte von 20 mm liegt das Nennflächengewicht bei 3000 g/m², wobei ferner eine Dichte von 150 kg/m³ zugrundeliegt. Durch Vorgabe der Materialdicke der Fasermatten kann problemlos eine Anpassung an die jeweiligen Einsatzbedingungen erfolgen und den Anforderungen hinsichtlich Wärmedämmung und/oder Schalldämmung entsprochen werden. Darüber hinaus sei festgehalten, dass die erfindungsgemäß zum Einsatz gelangenden Fasermatten über alle Temperaturbereiche ein sehr guten Schallabsorbtionsverhalten und eine sehr gute Vibrationsbeständigkeit aufweisen. Schließlich sei darauf hingewiesen, dass je nach den Dämmanforderungen die Gesamtdicke des Dämmelements im Bereich zwischen 5 bis 30 mm vorgegeben werden kann. Die Wärmeleitfähigkeit der Fasermatte ist in zweckmäßiger Welse derart vorgegeben, dass bei einer Temperatur von 100°C die Wärmeleitfähigkeit näherungsweise 0,033 W/mK beträgt, bei einer Temperatur von 300°C die Wärmeleltfähigkeit näherungsweise 0,056 W/mK beträgt, bei einer Temperatur von 500°C die Wärmeleitfähigkeit näherungsweise 0,0112 W/mK beträgt und bei einer Temperatur von 700°C die Wärmeleitfähigkeit 0,209 W/mK beträgt.

Besondere Ausgestaltungen und Weiterbildungen der Erfindung sind in den Unteransprüchen und der weiteren Beschreibung angegeben.

Die Erfindung wird nachfolgend anhand eines besonderen Ausführungsbeispiels näher erläutert, ohne dass insoweit eine Beschränkung erfolgt, Es zeigen:
- Fig. 1: schematisch einen Schnitt durch das Dämmelement,
- Fig. 2: vergrößert das Detail II gemäß Fig. 1,
- Fig. 3: ein Diagramm der Oberflächentemperatur über der Mediumstemperatur einer Versuchsreihe mit das Dämmelement enthaltenden Isolieraufbauten.

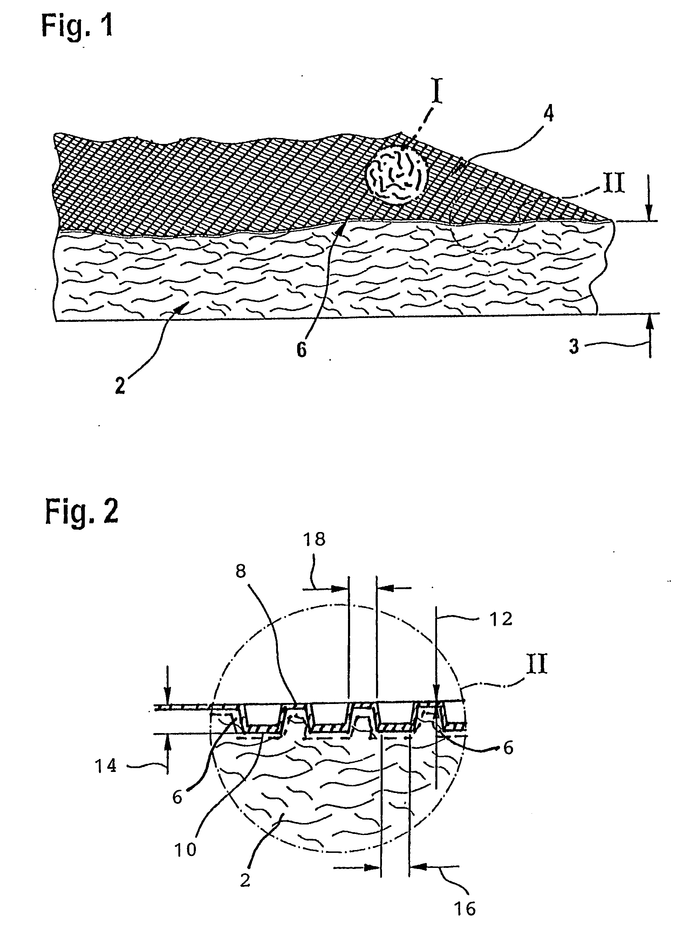

Fig. 1 zeigt teilweise und in einer perspektivischen Darstellung das Dämmelement, welches eine bevorzugt genadelte Fasermatte 2 enthält. Die Fasermatte 2 enthält zum einen Silikatfasern und zum anderen Basaltfasern gemäß den obigen Ausführungen. Bei dem hier dargestellten Ausführungsbeispiel besitzt die Fasermatte 2 eine Dicke 3 von im wesentlichen 20 mm. Es sei angemerkt, dass je nach Verwendung des Dämmelements die Gesamtdicke 3 zwischen 5 bis 30 mm betragen kann. Auf der gemäß Zeichnung oberen Seite ist die dünne mikrostrukturierte Edelstahlfolie 4 angeordnet, welche gemäß den obigen Erläuterungen ausgebildet ist. Zur Verbindung der auf die Fasermatte 2 kaschierten Edelstahlfolie 4 mit der Fasermatte 2 ist zwischen dieser und der Edelstahlfolie 4 ist das gleichfalls oben erläuterte, als Dispersionskleber ausgebildete Klebemittel 6 vorgesehen.

Die Mikrostrukturen sind als Täler oder Erhebungen in die zunächst plane Edelstahlfolie eingebracht, und zwar bevor die Edelstahlfolie mit dem Klebemittel auf die Fasermatte 2 aufgebracht bzw. kaschiert wurde. Wie durch gekreuzte Linien angedeutet, sind die Mikrostrukturen dicht nebeneinander und/oder mit vorgegebenen kleinen Abständen zueinander in der Edelstahlfolie vorgesehen. Alternativ können die Mikrostrukturen, wie durch das Detail I angedeutet, als unregelmäßige und/oder gekrümmte und/oder punktförmige und/oder kleine Flächenbereiche ausgebildete Mikrostrukturen sein. Unabhängig von der konkreten Ausbildung der Mikrostrukturen, seien es regelmäßig angeordnete oder unregelmäßig bzw. gekrümmt oder gepunktet ausgebildete Mikrostrukturen, sind im Rahmen der Erfindung die Mikrostrukturen über die Oberfläche der Edelstahlfolie mit vorgegebenen kleinen Abständen zueinander über die gesamte Oberfläche der Edelstahlfolie verteilt angeordnet.

Fig. 2 zeigt vergrößert das Detail II, wobei die Edelstahlfolie 4 die Mikrostrukturen 7 zum einen außen liegende Spitzenbereiche 8 und zum anderen innen liegende Talbereiche 10 aufweist. Bezogen auf die zunächst ebene Edelstahlfolie sind die Mikrostrukturen 7 als in Richtung nach innen zur Fasermatte 2 gerichtete Vertiefungen oder Täler ausgebildet. Alternativ können die Mikrostrukturen, und zwar wiederum auf die zunächst ebene Edelstahlfolie bezogen, als nach außen gerichtete Erhebungen ausgebildet sein. Sind gemäß Fig. 2 die Mikrostrukturen 7 als nach innen gerichtete Täler ausgebildet, so sind zwischen den benachbarten Mikrostrukturen 7 der Fasermatte 2 zugewandte Innenbereiche 11 vorhanden. Für den Fall von nach außen gerichteten Erhebungen liegen die Innenbereiche jeweils innerhalb der jeweiligen Mikrostruktur. Es ist von wesentlicher Bedeutung, dass die genannten Innenbereiche zumindest teilweise mit dem Klebemittel 6 ausgefüllt sind. Das Klebemittel 6 ist weiterhin teilweise in die der Edelstahlfolie zugewandte Außenseite der Fasermatte eingedrungen derart, dass dort eine Klebemittel enthaltende Grenzschicht vorhanden ist. Aufgrund der erfindungsgemäßen Mikrostrukturen 7 und/oder der Innenbereiche 11 ist folglich über das Klebemittel nicht nur eine reine Klebeverbindung, sondern zudem eine formschlüssige Verbindung der Edelstahlfolie 4 mit der Fasermatte 2 vorhanden. Die Mikrostrukturierung ist insbesondere durch Prägen der dünnen Edelstahlfolie 4 geschaffen, welche eine Materialdicke 12 von bevorzugt zumindest näherungsweise 0,05 mm aufweist. Zwischen den außen liegenden Spitzenbereichen 8 und den innen liegenden Talbereichen 10 ist, quer zur Mittelebene der Edelstahlfolie 4 gemessen, ein Abstand 14 vorgegeben, welcher erheblich größer als die Materialdicke 12 ist. Der Abstand 14, somit die Gesamthöhe der Folie 4 mit den Mikrostrukturen 7 ist überwiegend bevorzugt im Bereich zwischen 0,25 bis 0,36 mm, vorteilhaft zwischen 0,27 bis 0,33 mm und insbesondere im wesentlichen mit 0,3 mm vorgegeben. Ferner ist die Breite 16 der Mikrostrukturen 7 wesentlich größer als die Materialdicke 12 vorgegeben, bevorzugt im Bereich zwischen 0,7 bis 1,5 mm, vorteilhaft zwischen 0,8 bis 1,3 mm und insbesondere im wesentlichen mit 1 mm vorgegeben.

Weiterhin ist zwischen benachbarten Mikrostrukturen 7 ein Abstand 18 vorgegeben, welcher zweckmäßig größer als die Materialdicke 12 ist. In einer bevorzugten Ausgestaltung ist der Abstand 18 überwiegend im Bereich zwischen 0,15 bis 1,8 mm, vorteilhaft im Bereich zwischen 0,2 bis 1,4 mm und insbesondere im Bereich zwischen 0,3 bis 1 mm vorgegeben. Die Mikrostrukturen 7 weisen, und zwar gemäß Zeichnung im wesentlichen orthogonal zur Zeichenebene, vorgegebene Längen auf. Die Längen der Mikrostrukturen 7 betragen zweckmäßig mindestens 0,7 mm, vorteilhaft mindestens 0,8 mm und sind insbesondere mindestens 1 mm groß. Ferner weisen die Mikrostrukturen 7 eine vorgegebene maximale Länge auf, welche vorteilhaft um einen vorgegebenen Faktor größer ist als die Breite 16 der Mikrostrukturen 7. Bevorzugt ist dieser Faktor gleich 10, zweckmäßig 5 und insbesondere 3. Die Mikrostrukturierung kann als regelmäßige und sich unter vorgegebenen Winkeln kreuzende Linienstrukturen ebenso ausgebildet sein wie durch unregelmäßige und/oder gekrümmte Strukturbereiche, welche einerseits die außen liegenden Spitzenbereiche 8 und andererseits die innen liegenden Talbereiche 10 bilden. Aufgrund der erfindungsgemäß vorgesehenen Mikrostrukturierung ist trotz geringer Materialdicke 12 der Edelstahlfolie 4 zum einen deren Flexibilität verbessert und zum anderen deren Stabilität vor allem im Hinblick auf die thermische und/oder dynamische Belastung optimiert.

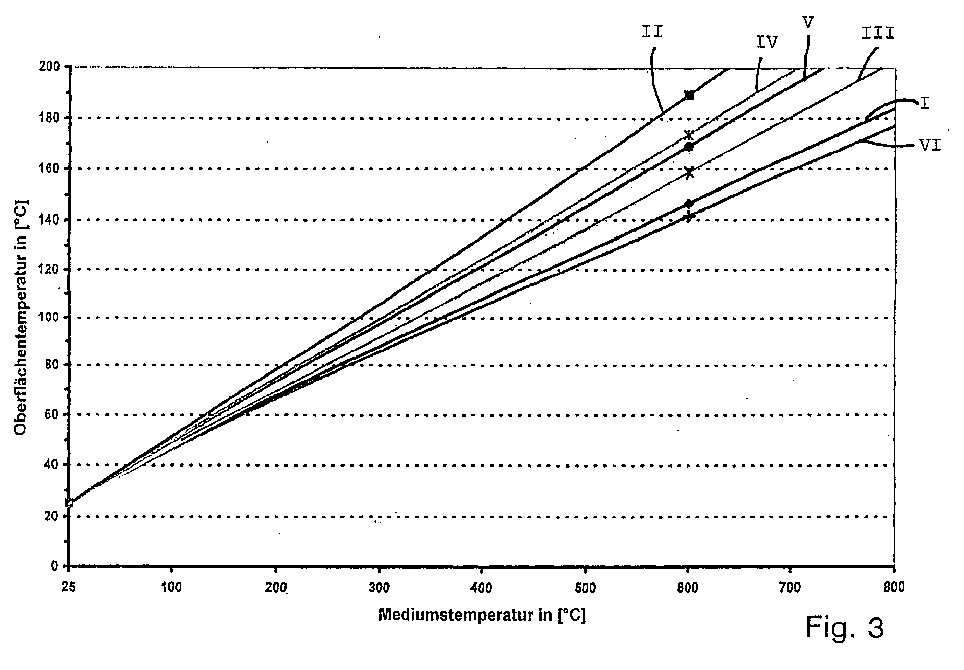

Fig. 3 zeigt ein Diagramm einer Versuchsreihe mit das Dämmelement enthaltenden Isolieraufbauten, wobei die Oberflächentemperatur über der Mediumstemperatur aufgetragen ist. Hierbei sind die Meßkurven mehrerer Varianten I bis VI aufgetragen, wobei die Variante V gemäß des bevorzugten Ausführungsbeispiels sich als besonders vorteilhaft erwiesen hat.

### Bezugszeichen

- 2: genadelte Fasermatte
- 3: Dicke von 2
- 4: mikrostrukturierte Edelstahlfolie
- 6: Klebemittel
- 7: Mikrostruktur / Prägung
- 8: außen liegender Spitzenbereich
- 10: innen liegender Talbereich
- 11: Innenbereich
- 12: Materialdicke von 4
- 14: Abstand zwischen 8 und 10 / Gesamthöhe von 4
- 16: Breite von 7
- 18: Abstand zwischen Mikrostrukturen

## Patentansprüche

1. Dämmelement, enthaltend eine strukturierte Edelstahlfolie (4) und Fasermaterial, wobei die Edelstahlfolie (4) und das als Fasermatte (2) ausgebildete Fasermaterial mittels eines Klebemittels (6) verbunden sind,
**dadurch gekennzeichnet, dass** die Fasermatte (2) nur auf einer Seite mit der einzigen, nebeneinander angeordnete Mikrostrukturen (7) aufweisenden Edelstahlfolie (4) kaschiert ist, deren Materialdicke (12) im Bereich zwischen 0,06 bis 0,15 mm vorgegeben ist, wobei die Gesamtdicke (3) des Dämmelements zwischen 5 bis 30 mm beträgt,
dass die Mikrostrukturen (7) als nach innen zur Fasermatte (2) gerichtete Talbereiche (10) oder nach außen gerichtete Spitzenbereiche (8) ausgebildet sind, wobei die Edelstahlfolie (4) auf ihrer der Fasermatte (2) zugewandten Innenseite Innenbereiche (11) aufweist, welche sich zwischen den Talbereichen (10) oder in den Spitzenbereichen (8) befinden,
dass der Abstand (18) zwischen benachbarten Mikrostrukturen (7) maximal 3 mm beträgt oder im Bereich zwischen 0,15 mm bis 3 mm vorgegeben ist,
dass das Klebemittel (6) als Hochtemperaturkleber ausgebildet ist, welcher eine Temperaturbeständigkeit bis zu 500°C aufweist und als ein nicht brennbarer und wasserfester Dispersionskleber ausgebildet ist,
und dass die Innenbereiche (11) mit dem Klebemittel (6) ausgefüllt sind, welches weiterhin teilweise in eine äußere Grenzschicht der Fasermatte (2) eingedrungen ist, wobei mittels des Klebemittels (6) zusätzlich zur Adhäsion eine zahnartige und formschlüssige Verbindung der Edelstahlfolie (4) mit der Fasermatte (2) vorhanden ist.

2. Dämmelement nach Anspruch 1, **dadurch gekennzeichnet, dass** die Edelstahlfolie (4) eine Materialdicke (12) aufweist, welche kleiner ist als die Abstände (14) zwischen außen liegenden Spitzenbereichen (8) und innen liegenden Talbereichen (10) der Mikrostrukturen (7) der Edelstahlfolie (4), wobei die Materialdicke (12) der Edelstahlfolie (4) im Bereich zwischen 0,07 bis 0.13 mm oder im Bereich zwischen 0.09 bis 0.12 mm vorgegeben ist.

3. Dämmelement nach Anspruch 1 oder 2, **dadurch gekennzeichnet, dass** die Edelstahlfolie (4) aus einer Eisen-Chrom-Nickel-Legierung besteht und/oder dass in Gew.% der Anteil von Eisen bei 50 bis 80%, der Anteil von Chrom zwischen 10 bis 28% und der Nickelanteil zwischen 5 bis 25% vorgegeben ist, wobei der Anteil von Eisen zwischen 52 bis 78%, der Anteil von Chrom zwischen 12 bis 24% und der Anteil von Nickel zwischen 6 bis 19% vorgegeben ist.

4. Dämmelement nach einem der Ansprüche 1 bis 3, **dadurch gekennzeichnet, dass** die flexible Fasermatte (2) als eine Fasernadelmatte ausgebildet ist und/oder dass die Fasermatte (2) ein Gemisch aus Glasfasern und Mineralfasern enthält und/oder dass die Fasermatte (2) sowohl Silikatfasern als auch Basaltfasern enthält, wobei die Fasern der Fasermatte (2) oder deren Silikatfasern, eine Faserstärke im Mikrometerbereich oder im Bereich zwischen 10 bis 17 µm aufweisen.

5. Dämmelement nach Anspruch 4, **dadurch gekennzeichnet, dass** der Anteil von Silikatfasern im Bereich zwischen 20 bis 40% oder 30% beträgt und/oder dass der Anteil der Basaltfasern im Bereich zwischen 80 bis 60%, näherungsweise von 70% vorgegeben ist.

6. Dämmelement nach einem der Ansprüche 1 bis 5, **dadurch gekennzeichnet, dass** die Wärmeleitfähigkeit der Fasern der Fasermatte (2) bei einer Temperatur von 100°C näherungsweise bei 0,033 W/mK liegt, bei einer Temperatur von 300°C näherungsweise bei 0,056 W/mK, bei einer Temperatur von 500°C näherungsweise bei 0,0112 W/mK und bei einer Temperatur von 700°C bei 0,209 W/mk und/oder dass die Fasermatte (2) oder deren Fasern eine hohe Temperaturbeständigkeit bis 750°C aufweisen und/oder dass die Fasermatte (2) hoch rüttelfest ausgebildet ist.

7. Dämmelement nach einem der Ansprüche 1 bis 6, **dadurch gekennzeichnet, dass** die Gesamthöhe bzw. der Abstand (14) zwischen außen liegenden Spitzenbereichen (8) und innenliegenden Talbereichen (10) der Mikrostrukturen (7) um einen vorgegebenen Faktor größer ist als die Materialdicke (12) oder im Bereich zwischen 0,06 bis 0,15 mm oder im Bereich zwischen 0,07 bis 0,13 mm oder im Bereich zwischen 0,09 und 0,12 mm, und/oder dass die Breite der Mikrostrukturen (7) im Bereich zwischen 0,7 bis 1,5 mm oder im Bereich zwischen 0,8 bis 1,3 mm oder mit 1 mm vorgegeben sind.

8. Dämmelement nach einem der Ansprüche 1 bis 7, **dadurch gekennzeichnet, dass** der Abstand (18) zwischen benachbarten Mikrostrukturen (7) 2 mm oder 1 mm beträgt, und/oder dass der Abstand (18) zwischen benachbarten Mikrostrukturen (7) zwischen 0,2 bis 1,4 mm oder im Bereich zwischen 0,3 bis 1 mm vorgegeben ist.

9. Dämmelement nach einem der Ansprüche 1 bis 8, **dadurch gekennzeichnet, dass** die Länge der Mikrostrukturen (7) 0,7 mm oder 0,8 mm oder 1 mm beträgt und/oder dass die Mikrostrukturen (7) maximale Längen aufweisen, welche um einen vorgegebenen Faktor größer als die Breite (16) der Mikrostrukturen (7) sind, wobei der Faktor zehnmal so groß oder fünfmal so groß oder dreimal so groß wie die jeweilige Breite (16) der Mikrostrukturen (7) ist.

## Claims

1. Insulating element containing a structured stainless steel foil (4) and fibrous material, wherein the stainless steel foil (4) and the fibrous material configured as a fibre mat (2) are connected by means of an adhesive (6), **characterised in that** the fibre mat (2) is laminated only on one side with the single stainless steel foil (4) having adjacently disposed microstructures (7), whose material thickness (12) is predefined in the range between 0.06 to 0.15 mm, wherein the total thickness (3) of the insulating element is between 5 to 30 mm, that the microstructures (7) are configured as trough regions (10) directed inwardly to the fibre mat (2) or outwardly directed peak regions (8), wherein the stainless steel foil (4) has inner regions (11) on its inner side facing the fibre mat (2) which are located between the trough regions (10) or in the peak regions (8), that the distance (18) between adjacent microstructures (7) is a maximum of 3 mm or is predefined in the range between 0.15 mm to 3 mm, that the adhesive (6) is configured as high-temperature adhesive which has a temperature resistance up to 500°C and is configured as a non-flammable and waterproof dispersion adhesive, and the inner regions (11) are filled with the adhesive (6) which furthermore has partially penetrated into an outer boundary layer of the fibre mat (2), wherein by means of the adhesive (6) in addition to the adhesion a tooth-like and positive connection of the stainless steel foil (4) with the fibre mat (2) is provided.

2. The insulating element according to claim 1, **characterised in that** the stainless steel foil (4) has a material thickness (12) which is less than the distances (14) between outer-lying peak regions (8) and inner-lying trough regions (10) of the microstructures (7) of the stainless steel foil (4), wherein the material thickness (12) of the stainless steel foil (4) is predefined in the range between 0.07 to 0.13 mm or in the range between 0.09 to 0.12 mm.

3. The insulating element according to claim 1 or 2, **characterised in that** the stainless steel foil (4) consists of an iron-chromium-nickel alloy and/or that in wt.% the fraction of iron is predefined as 50 to 80%, the fraction of chromium is predefined as between 10 to 28% and the nickel fraction is predefined as between 5 to 25%, wherein the fraction of iron is predefined between 52 to 78%, the fraction of chromium is predefined between 12 to 24% and the fraction of nickel is predefined between 6 to 19%.

4. The insulating element according to any one of claims 1 to 3, **characterised in that** the flexible fibre mat (2) is configured as a fibre needle mat and/or the fibre mat (2) contains a mixture of glass fibres and mineral fibres and/or that the fibre mat (2) contains both silicate fibres and also basalt fibres, wherein the fibres of the fibre mat (2) or the silicate fibres thereof have a fibre thickness in the micrometre range or in the range between 10 to 17 µm.

5. The insulating element according to claim 4, **characterised in that** the fraction of silicate fibres is in the range between 20 to 40% or 30% and/or that the fraction of basalt fibres is predefined in the range between 80 to 60%, approximately 70%.

6. The insulating element according to any one of claims 1 to 5, **characterised in that** the thermal conductivity of the fibres of the fibre mat (2) at a temperature of 100°C lies at approximately 0.033 W/mK, at a temperature of 300°C at approximately 0.056 W/mK, at a temperature of 500°C at approximately 0.0112 W/mK and at a temperature of 700°C at 0.209 W/mK and/or that the fibre (2) or the fibres thereof has a high temperature resistance up to 750°C and/or that the fibre mat (2) is configured to be highly vibrationproof.

7. The insulating element according to any one of claims 1 to 6, **characterised in that** the total height or the distance (14) between outer-lying peak regions (8) and inner-lying trough regions (10) of the microstructures (7) is a predefined factor greater than the material thickness (12) or is predefined in the range between 0.06 to 0.15 mm or in the range between 0.07 to 0.13 mm or in the range between 0.09 and 0.12 mm and/or that the width of the microstructures (7) is predefined in the range between 0.07 to 1.5 mm or in the range between 0.8 to 1.3 mm or 1 mm.

8. The insulating element according to any one of claims 1 to 3, **characterised in that** the distance (18) between adjacent microstructures (7) is 2 mm or 1 mm and/or that the distance (18) between adjacent microstructures (7) is predefined between 0.2 to 1.4 mm or in the range between 0.3 to 1 mm.

9. The insulating element according to any one of claims 1 to 8, **characterised in that** the length of the microstructures (7) is 0.7 mm or 0.8 mm or 1 mm and/or that the microstructures (7) have maximum lengths which are a predefined factor greater than the width (16) of the microstructures (7), wherein the factor is ten times as great or five times as great or three times as great as the respective width (16) of the microstructures (7).

## Revendications

1. Elément isolant, contenant un film en acier inox structuré (4) et du matériau fibreux, le film en acier inox (4) et le matériau fibreux se présentant sous forme de natte fibreuse (2) étant reliés au moyen d'une colle (6),
**caractérisé en ce que** la natte fibreuse (2) n'est plaquée que sur une face avec l'unique film en acier inox (4) présentant des microstructures juxtaposées (7) dont l'épaisseur de matériau (12) est prédéfinie dans la plage de 0,06 à 0,15 mm, l'épaisseur totale (3) de l'élément isolant allant de 5 à 30 mm,
que les microstructures (7) se présentent sous forme de sous-parties (10) orientées vers l'intérieur vers la natte fibreuse (2) ou de parties effilées (8) orientées vers l'extérieur, le film en acier inox (4) présentant sur sa face interne tournée vers la natte fibreuse (2) des parties internes (11) qui se trouvent entre les sous-parties (10) ou dans les parties effilées (8),
que la distance (18) entre les microstructures voisines (7) est au maximum de 3 mm ou est prédéfinie dans la plage de 0,15 mm à 3 mm,
que la colle (6) se présente sous forme d'une colle à haute température qui présente une résistance thermique allant jusqu'à 500°C et se présente sous forme d'une colle à dispersion ininflammable et résistante à l'eau
et que les parties internes (11) sont comblées par la colle (6) qui a pénétré partiellement plus loin dans une couche limite extérieure de la natte fibreuse (2), une liaison au moyen de la colle (6) par correspondance géométrique de type dents du film en acier inox (4) avec la natte fibreuse (2) étant prévue en plus pour assurer l'adhérence.

2. Elément isolant selon la revendication 1, **caractérisé en ce que** le film en acier inox (4) présente une épaisseur de matériau (12) qui est inférieure aux distances (14) entre les parties effilées situées à l'extérieur (8) et les sous-parties situées à l'intérieur (10) des microstructures (7) du film en acier inox (4), l'épaisseur de matériau (12) du film en acier inox (4) étant prédéfinie dans la plage de 0,07 à 0,13 mm ou dans la plage de 0,09 à 0,12 mm.

3. Elément isolant selon la revendication 1 ou 2, **caractérisé en ce que** le film en acier inox (4) est composé d'un alliage de fer-chrome-nickel et/ou que, en pourcentage du poids, la part du fer est prédéfinie entre 50 et 80 %, la part du chrome entre 10 et 28 % et la part du nickel entre 5 et 25 %, la part du fer étant prédéfinie entre 52 et 78 %, la part du chrome entre 12 et 24 % et la part du nickel entre 6 et 19 %.

4. Elément isolant selon une des revendications 1 à 3, **caractérisé en ce que** la natte fibreuse souple (2) se présente sous forme d'une natte fibreuse aiguilletée et/ou que la natte fibreuse (2) contient un mélange de fibres de verre et de fibres minérales et/ou que la natte fibreuse (2) contient aussi bien des fibres de silicate que des fibres de basalte, les fibres de la natte fibreuse (2) ou ses fibres de silicate présentant une épaisseur de fibres de l'ordre du micromètre ou dans la plage de 10 à 17 µm.

5. Elément isolant selon la revendication 4, **caractérisé en ce que** la part des fibres de silicate est prédéfinie entre 20 et 40 % ou s'élève à 30 % et/ou que la part des fibres de basalte est prédéfinie entre 80 et 60 %, approximativement à 70 %.

6. Elément isolant selon une des revendications 1 à 5, **caractérisé en ce que** la conductivité thermique des fibres de la natte fibreuse (2) à une température de 100°C se situe approximativement à 0,033 W/mK, à une température de 300°C approximativement à 0,056 W/mK, à une température de 500°C approximativement à 0,0112 W/mK et à une température de 700°C approximativement à 0,209 W/mK et/ou que la natte fibreuse (2) ou ses fibres présentent une résistance thermique élevée allant jusqu'à 750°C et/ou que la natte fibreuse (2) est conçue pour bien résister aux secousses.

7. Elément isolant selon une des revendications 1 à 6, **caractérisé en ce que** la hauteur totale ou la distance (14) entre les parties effilées (8) situées à l'extérieur et les sous-parties internes (10) des microstructures (7) est supérieure à raison d'un facteur prédéfini à l'épaisseur de matériau (12) ou est prédéfinie dans la plage de 0,06 à 0,15 mm ou dans la plage de 0,07 à 0,13 mm ou dans la plage de 0,09 à 0,12 mm et/ou la largeur des microstructures (7) dans la plage de 0,7 à 1,5 mm ou dans la plage de 0,8 à 0,13 mm ou à 1 mm.

8. Elément isolant selon une des revendications 1 à 7, **caractérisé en ce que** la distance (18) entre les microstructures voisines (7) est de 2 mm ou 1 mm et/ou que la distance (18) entre les microstructures voisines (7) est prédéfinie entre 0,2 et 1,4 mm ou dans la plage de 0,3 à 1 mm.

9. Elément isolant selon une des revendications 1 à 8, **caractérisé en ce que** la longueur des microstructures (7) est de 0,7 mm ou 0,8 mm ou 1 mm et/ou que les microstructures (7) présentent des longueurs maximales qui sont supérieures à raison d'un facteur prédéfini à la largeur (16) des microstructures (7), le facteur étant dix fois plus élevé ou cinq fois plus élevé ou trois fois plus élevé que la largeur respective (16) des microstructures (7).
